# EUROPÄISCHE PATENTSCHRIFT

(11) **EP 0 403 909 B1**
(45) Veröffentlichungstag und Bekanntmachung des Hinweises auf die Patenterteilung: **06.04.1994**
(21) Anmeldenummer: 90110993.4
(22) Anmeldetag: 11.06.1990
(51) Int. Cl.: C07C 49/175, C07C 45/50, C07C 47/198

(54) **Verfahren zur Herstellung von 2,3-Dialkoxypropanalen**
Method for the production of 2,3-dialkoxypropanals
Procédé pour la fabrication de dialcoxy-2,3-propanals

(30) Priorität: 22.06.1989 DE 3920423
(43) Veröffentlichungstag der Anmeldung: 27.12.1990
(73) Patentinhaber: BASF Aktiengesellschaft, 67063 Ludwigshafen (DE)
(72) Erfinder: Koeffer, Dieter, Dr., D-6940 Weinheim (DE); Maerkl, Robert, Dr., D-6701 Fussgoenheim (DE); Bertleff, Werner, Dr., D-6806 Viernheim (DE)

(56) Entgegenhaltungen:
- EP-A- 0 024 532
- US-A- 4 533 756
- CHEMICAL ABSTRACTS, Band 98, Nr. 15, 11. April 1983, Seite 678, Abstract Nr. 126494f, Columbus, Ohio, US; M. PETRUSOVA et al, "Preparation of 2,3-di-O-methylglyceraldehyde and its beta-elimination product 2-methoxypropenal"
- Index Band 98, 1983, Seite 5560CS, Spalte 3, Zeilen 44-48 (Kat. D)
- Polymer Journal Vol.2, No. 2, 153-160 (1971)

## Beschreibung

Die Vorliegende Erfindung betrifft ein verfahren zur Herstellung von 2,3-Dialkoxypropanalen der allgemeinen Formel I 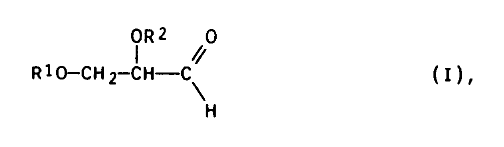
in der R¹ und R² C₁- bis C₈-Alkylgruppen oder C₅- bis C₆-Cycloalkylgruppen bedeuten, die ihrerseits Substituenten tragen können, welche sich unter den Reaktionsbedingungen inert verhalten.

2,3-Dialkoxypropanale waren bislang nur aus Kohlenhydrat-Derivaten erhältlich. Beispielsweise konnte das einfachste Glied dieser Verbindungsklasse, 2,3-Dimethoxypropanal, bisher nur entweder durch Oxidation von 1,2,5,6-Tetra-O-methylmannit mit Blei(IV)acetat (Baer et al., J. Biol. Chem. 145, 61 (1942)) oder Natriumperiodat (Fedoronko et al., Carbohyd. Res. 87, 51 (1980)) oder alternativ hierzu aus Glycerinaldehyddimethylacetal nach Veretherung mit Methyljodid/Natriumhydrid und anschließender Acetalhydrolyse (Chem. Zvesti 36, 837 (1982)) hergestellt werden. Diese Verfahren sind allerdings nur für den Labormaßstab geeignet, da schon die Herstellung der Ausgangsverbindungen mehrstufige Synthesen erfordert.

Obwohl die Herstellung von Aldehyden mit Hilfe der Hydroformylierungsreaktion ein vielseitig genutztes technisches Verfahren ist, ist über die Hydroformylierung von 1,2-Dialkoxyethenen bisher nichts bekannt (vgl. Botteghi et al., J. Mol. Cat. 40, 129-182 (1987), insbesondere S. 147, Kap. 3.2.). Hingegen ist die Hydroformylierung von Vinylethern durch Lin et al. (US-A-4 533 756) beschrieben worden. Snapp et al. (US-A-3 888 880) berichten lediglich über die sehr spezielle Synthese von Dioxancarbaldehyd durch die Kobaltnaphthenat-katalysierte Hydroformylierung von Dioxen.

Da 2,3-Dialkoxypropanale vielseitig verwendbare Synthesebausteine sind und außerdem aus ihnen durch basenkatalysierte Eliminierung von Alkohol 2-Alkoxyacroleine gewonnen werden können, welche ihrerseits als Monomere und Synthesebausteine breite Verwendung finden, lag der Erfindung die Aufgabe zugrunde, ein Verfahren zu finden, das es erlaubt, 2,3-Dialkoxypropanale im industriellen Maßstab auf einfache und wirtschaftliche Weise herzustellen.

Dementsprechend wurde ein Verfahren zur Herstellung von 2,3-Dialkoxypropanalen der allgemeinen Formel I 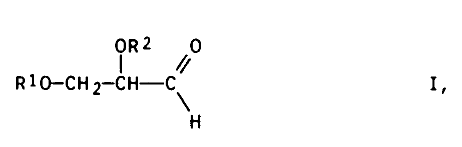
in der R¹ und R² C₁- bis C₈-Alkylgruppen oder C₅- bis C₆-Cycloalkylgruppen bedeuten, die ihrerseits Substituenten tragen können, welche sich unter den Reaktionsbedingungen inert verhalten, gefunden, das dadurch gekennzeichnet ist, daß man ein 1,2-Dialkoxyethen der allgemeinen Formel II

R¹O-HC=CH-OR² II,

mit Kohlenmonoxid und Wasserstoff in Gegenwart eines Rhodium-haltigen Katalysators umsetzt.

Die Reste R¹ und R² können unabhängig voneinander für C₁- bis C₈-Alkyl- und/oder C₅-6₆-Cycloalkylgruppen stehen. Gegebenenfalls können die Gruppen R¹ und R2 1 bis 2 Substituenten tragen, welche sich unter den Reaktionsbedingungen inert verhalten, beispielsweise Alkoxy-, Acetal- oder Estergruppen. Bevorzugt stehen R¹ und R² jedoch für unsubstituierte C₁-bis C₈-Alkyl- oder C₅- C₆-Cycloalkylgruppen, wobei von den Alkylgruppen C₁-bis C₄-Alkylgruppen besonders bevorzugt sind. Insonderheit stehen R¹ und R² beispielsweise für die Methyl-, Ethyl-, Propyl-, Isopropyl-, Butyl-, Isobutyl-, tert. Butyl-, Pentyl-, Hexyl-, Heptyl-, Octyl-, 2-Ethylhexyl-, Cyclopentyl- oder die Cyclohexylgruppe.

Als Ausgangsmaterial zur Herstellung der 2,3-Dialkoxypropanale I werden die mit den entsprechenden Gruppen R¹ und R² substituierten Dialkoxyethene II verwendet, welche auf einfache Weise durch die Magnesium-katalysierte Chloreliminierung aus 1,2-Dialkoxy-1,2-dichlorethanen (vgl. Baganz et al., Chem. Ber. 96, 2657 (1963)) oder aus 1,1,2-Trialkoxyethanen mittels der Bariumhydroxid/Kieselgel-(US-A 2 479 068) oder der Zeolith-katalysierten (EP-A 302 486) Alkoholabspaltung in der Gasphase zugänglich sind.

Zur Herstellung der 2,3-Dialkoxypropanale I werden die Dialkoxyethene II mit einem Kohlenmonoxid/Wasserstoff-Gasgemisch in Gegenwart eines Rhodium-haltigen Katalysators umgesetzt. Als Rhodium-haltige Katalysatoren können Rhodiumverbindungen wie sie in US-A-28 80 241 beschrieben sind, insbesondere Rhodiumsalze, wie Rhodiumacetat oder Rhodiumacetonylacetonat, Rhodiumoxide und Rhodiumcarbonylverbindungen verwendet werden. Besonders bevorzugt werden die 2,3-Dialkoxypropanale mit Hilfe eines Katalysatorsystems aus Rhodium und einer tertiären Phosphorverbindung hergestellt. Derartige zur Durchführung von Hydroformylierungen geeignete Katalysatorsysteme sind Stand der Technik, beispielsweise können die in DE-A-17 93 069 beschriebenen Katalysatorsysteme aus Rhodium und tertiären Phosphorverbindungen im erfindungsgemäßen Verfahren eingesetzt werden. Bevorzugt werden solche Katalysatorsysteme eingesetzt, in denen die tertiäre Phosphorverbindung ein Triarylphosphin oder ein Triarylphosphit ist, beispielsweise Triphenylphosphin, Tritolylphosphin, Triphenylphosphit und Tritolylphosphit.

Als Katalysatorsystem Rhodium/tertiäre Phosphorverbindung können Rhodiumkomplexe Verwendung finden wie sie in DE-A 17 93 069 genannt sind. Das Katalysatorsystem kann aber auch vorteilhaft in situ, d.h. im Reaktionsgemisch der Hydroformylierung und in Gegenwart von Wasserstoff und Kohlenmonoxid, aus einer Rhodiumquelle und einer tertiären Phosphorverbindung hergestellt werden. Als Rhodiumquelle können beispielweise Rhodiumsalze, Rhodiumcarbonylverbindungen oder Rhodiumoxide verwendet werden. Die tertiäre Phosphorverbindung wird im allgemeinen bezüglich des Rhodiums in einem 1- bis 300-fachen molaren überschuß, vorzugsweise in einem Molverhältnis von 3 : 1 bis 200 : 1 verwendet.

Die Hydroformylierung der Dialkoxyethene II kann in Gegenwart von unter den Reaktionsbedingungen inerten Lösungsmitteln durchgeführt werden. Als Lösungsmittel sind beispielsweise Ether, wie Diphenylether oder Dioxan, Acetale, wie 1,3-Dioxan, Ester, wie Dibutylphthalat, sowie aliphatische oder aromatische Kohlenwasserstoffe, wie Hexan, Cyclohexan oder Toluol, geeignet.

Im allgemeinen geht man bei der Durchführung der Umsetzung so vor, daß man das Dialkoxyethen II zusammen mit den Komponenten zur Herstellung des Rhodium-Katalysators und ggf. zusammen mit einem Lösungsmittel in einem Autoklaven vorlegt und auf diesen Reaktionsansatz bei Temperaturen von 50 bis 200°C, bevorzugt bei 60 bis 180°C, insbesondere bei 80 bis 150°C das Kohlenmonoxid/Wasserstoff-Gemisch mit einem Druck von im allgemeinen 2 bis 300 bar, insbesondere von 5 bis 100 bar, aufpreßt. Das Molverhältnis Kohlenmonoxid/Wasserstoff kann weitgehend frei gewählt werden. Zweckmäßigerweise beträgt das Kohlenmonoxid/Wasserstoff-Molverhältnis jedoch 0,1 bis 2. Besonders bevorzugt werden solche Kohlenmonoxid/Wasserstoff-Gasgemische verwendet, wie sie bei der Herstellung von Synthesegas entstehen.

Die Reaktion kann sowohl diskontinuierlich als auch kontinuierlich im Rohrreaktor erfolgen. Die 2,3-Dialkoxypropanale können aus dem Reaktionsaustrag durch Destillation isoliert werden.

### Beispiele

### Beispiel 1

Ein 300-ml-Autoklav wurde mit 0,56 mol 1,2-Dimethoxyethen, 40 g Cyclohexan, 0,2 mmol Hydridocarbonyltris(triphenylphosphin)rhodium und 20 mmol Triphenylphosphin beschickt. Bei Raumtemperatur wurde mit einem Kohlenmonoxid/Wasserstoff-Gasgemisch (CO/H₂-Molverhältnis: 1/1) ein Druck von 20 bar eingestellt.

Das Reaktionsgemisch wurde im Autoklaven auf 130°C aufgeheizt und der Druck durch Nachpressen des CO/H₂-Gasgemisches auf 70 bar erhöht. Nach 3 h Reaktionszeit wurde der Autoklav abgekühlt und entspannt, und aus dem flüssigen Reaktionsaustrag das 2,3-Dimethoxypropanal durch fraktionierende Destillation isoliert.
Ausbeute: 61 %.

### Beispiel 2

Ein 300-ml-Autoklav wurde mit 1,1 mol 1,2-Dimethoxyethen und 0,08 mmol (Acetylacetonato)dicarbonylrhodium beschickt. Bei Raumtemperatur wurde mit einem Kohlenmonoxid/Wasserstoff-Gasgemisch (vgl. Beispiel 1) ein Druck von 20 bar eingestellt.

Das Reaktionsgemisch wurde auf eine Temperatur von 100°C erhitzt, der Druck im Autoklaven durch Nachpressen des Kohlenmonoxid/Wasserstoff-Gasgemisches auf 280 bar erhöht und für eine Reaktionszeit von 8 h auf diesem Niveau gehalten. 2,3-Dimethoxypropanal war im Reaktionsaustrag (127,8 g) zu 82,5 Gew.-% enthalten (GC-Analyse).

## Patentansprüche

1. Verfahren zur Herstellung von 2,3-Dialkoxypropanalen der allgemeinen Formel I 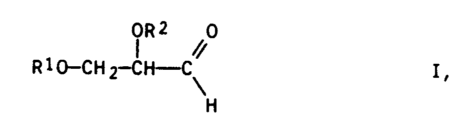 in der R¹ und R² C₁- bis C₈-Alkylgruppen oder C₅- bis C₆-Cycloalkylgruppen bedeuten, die ihrerseits Substituenten tragen können, welche sich unter den Reaktionsbedingungen inert verhalten, dadurch gekennzeichnet, daß man ein 1,2-Dialkoxyethen der allgemeinen Formel II
R¹O-HC=CH-OR² II,
mit Kohlenmonoxid und Wasserstoff in Gegenwart eines Rhodium-haltigen Katalysators umsetzt.

2. Verfahren nach Anspruch 1, dadurch gekennzeichnet, daß man die Umsetzung in Gegenwart eines Katalysatorsystems aus Rhodium und einer tertiären Phosphorverbindung durchführt.

3. Verfahren nach Anspruch 2, dadurch gekennzeichnet, daß das Molverhältnis der tertiären Phosphorverbindung zu Rhodium 3 : 1 bis 200 : 1 beträgt.

4. Verfahren nach den Ansprüchen 2 oder 3, dadurch gekennzeichnet, daß man als tertiäre Phosphorverbindung ein Triarylphosphin oder Triarylphosphit verwendet.

## Claims

1. A process for the preparation of a 2,3-dialkoxypropanal of the formula I 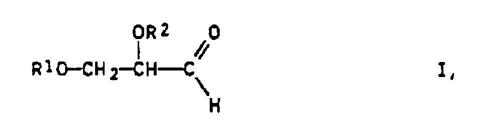 where R¹ and R² are each C₁-C₈-alkyl or C₅- or C₆-cycloalkyl, which in turn may carry substituents which are inert under the reaction conditions, wherein a 1,2-dialkoxyethene of the formula II
R¹⁰-HC=CH-OR² II
is reacted with carbon monoxide and hydrogen in the presence of a rhodium-containing catalyst.

2. A process as claimed in claim 1, wherein the reaction is carried out in the presence of a catalyst system comprising rhodium and a tertiary phosphorus compound.

3. A process as claimed in claim 2, wherein the molar ratio of the tertiary phosphorus compound to rhodium is from 3 : 1 to 200 : 1.

4. A process as claimed in claim 2 or 3, wherein the tertiary phosphorus compound used is a triarylphosphine or triaryl phosphite.

## Revendications

1. Procédé de préparation de 2,3-dialcoxypropanals de la formule générale I 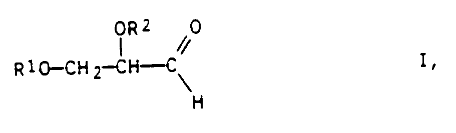 dans laquelle R¹ et R² représentent des radicaux alkyle en C₁ à C₈, ou cycloalkyle en c₅ ou C₆, qui, à leur tour, portent des substituants, qui se comportent de manière inerte dans les conditions réactionnelles, caractérisé en ce que l'on fait réagir un 1,2-dialcoxyéthène de la formule II
R¹O-HC=CH-OR² II,
avec du monoxyde de carbone et de l'hydrogène en présence d'un catalyseur contenant du rhodium.

2. Procédé suivant la revendication 1, caractérisé en ce que l'on entreprend la réaction en présence d'un système catalytique constitué de rhodium et d'un composé du phosphore tertiaire.

3. Procédé suivant la revendication 2, caractérisé en ce que le rapport molaire du composé du phosphore tertiaire au rhodium varie de 3:1 à 200:1.

4. Procédé suivant la revendication 2 ou 3, caractérisé en ce que l'on utilise une triarylphosphine ou un phosphite de triaryle à titre de composé du phosphore.
